# EUROPEAN PATENT APPLICATION

(11) **EP 2 292 296 A1**
(43) Date of publication of application: **09.03.2011**
(21) Application number: 10175695.5
(22) Date of filing: 08.09.2010
(51) Int. Cl.: A61M 39/02, A61M 39/04, A61M 39/26

(54) **Swabbable needleless valve**

(30) Priority: 08.09.2009 TR 200906911
(71) Applicant: Asset Medikal Tasarim Sanayi Ve Ticaret Anonim Sirketi, Istanbul (TR)
(72) Inventor: Tuysuz, Mehmet, ISTANBUL (TR)
(74) Representative: Dericioglu, Ekin

(57) **Abstract**

The present invention relates to a swabbable needleless valve (1) which is used for closing the end of the intravenous catheter for vascular access which extends out of the body, prevents the medical staff from contacting with the blood of the patient, has a swabbable insert surface and essentially comprises a body (5) which has an insert side (2) into which the male luer (M) of the syringe and serum sets used in the treatment are inserted, an exit side (3) which enables to connect to the catheter and a connective region (4) which connects these two portions (2, 3) to each other, and a valve member (6) which enables control of the fluid passage.

## Description

### Field of the Invention

The present invention relates to a swabbable needleless valve which is used for closing the end of the intravenous catheter for vascular access which extends out of the body, prevents the medical staff from contacting with the blood of the patient, has a swabbable insert surface and is suitable for the needleless fluid exchange.

### Prior Art

In the field of medicine, the first treatment applied to a patient is to place an intravenous (IV) catheter for vascular access. The intravenous (IV) catheter consists of a fine plastic tubing which is inserted into the vein of a patient having a diameter of 1-1,5 mm, and a length of 20-45 mm, and a connector which is placed on this fine tubing which extends out of the body. The connector is in the form of a female luer. Said female luer is for connection to the male luer of other medical devices such as sterile syringe, serum set, expansion line which are used to give medicine and fluid to the patient.

The end in the form of fine tubing of the intravenous catheter (distal end) is inserted into the vein of the patient by means of a guiding needle passing through the catheter. Then, the needle is removed from inside of the catheter and disposed. The open end with connector (the proximal end) of the catheter which extends out of the body is closed by a cap. Depending on the course of the treatment, the cap is removed and fluid-medicament is given to the patient, blood sample is taken where necessary and then the cap is put back again.

In the conventional application, after the intravenous catheter is placed into the vein, the needle inside of the catheter is removed and disposed, and the end of the catheter is closed by a standard cap. When it is necessary to administer medicament-fluid to the patient and to take blood sample from the patient, this cap is removed and fluid exchange is implemented. Following this process, the cap is put back on the same place again. There is probability of touching blood of the patient for the medical staff with his/her hand whenever the cap is opened. Moreover, there is a risk of contamination of the cap with the infectious agents in the environment and transmission of these infectious agents to the vein access of the patient when the cap is put back again.

In order to reduce the opening and closing frequency of the cap and to reduce thereby the risk of contamination (infection with the microorganism), caps with latex membrane have been used instead of conventional standard caps. The caps with latex membrane are opened less frequently during the treatment, which partially protects the patient against the contagious external agents. However, the requirement for using the latex membrane with a piercing needle cannot bring solution for the needle stick accidents. Additionally, the latex caps are required to be removed in order to connect a serum set to the patient.

The swabbable valves are used to close the end of the intravenous catheter which extends out of the body of a patient as a separate medical device. A normally closed valve, this valve substituting the conventional cap enables disinfection of the insert surface by wiping and fluid exchange without using a needle. The valve comes into open position by activation of stub male luer of other medical devices.

The first prominent examples of the valve concept enabling fluid exchange without using needle are disclosed in the Italian Patent Document No. 22356/74 (US Patent No:3,994,293) by Antonio Ferro in 1974, and in the Federal German Republic Patent Document No. 2817102 (US Patent No:4,387,879) by Stefan Tauschinski in 1978.

The main principle of the valves access surface of which can be wiped is that the access surface of the valve is always in the closed position, fluid pathway is opened in both directions by inserting the male luer of another medical device into the valve, and is closed again so as not to allow contact with the environment when the male luer is removed.

In the state of the art, examples for the swabbable valve concept include US Patent No: 5,676,346, US Patent No: 5,699,821, US Patent No: 6,036,171, US Patent No: 6,651,956, US Patent No: 6,039,302, US Patent No: 6,079,432.

### Summary of the Invention

The objective of the present invention is to realize a swabbable needleless valve which enables to essentially meet the safety need of patient and medical staff.

Another objective of the present invention is to realize a swabbable needleless valve which enables to provide manufacture and assembling facilities.

A further objective of the present invention is to realize a cost efficient swabbable needleless valve.

Yet another objective of the present invention is to realize a swabbable needleless valve which is ergonomic and easy to use.

An additional objective of the present invention is to realize a swabbable needleless valve which enables the total treatment cost to be reduced.

### Detailed Description of the Invention

"Swabbable needleless valve" realized to fulfill the objective of the present invention is illustrated in the accompanying figures wherein,
Figure 1 is cross-section view of the inventive swabbable needleless valve.
Figure 2 is cross-section view of another embodiment according to the inventive swabbable needleless valve.
Figure 3 is cross-section view of an embodiment according to the inventive swabbable needleless valve in which the male luer is inserted.
Figure 4 is cross-section view of flow cut-off member in the state of being displaced in an embodiment according to the inventive swabbable needleless valve.
Figure 5 is an isometric view of the valve member used in the inventive swabbable needleless valve.
Figure 6 is a cross sectional view of the valve member used in the inventive swabbable needleless valve.
Figure 7 is a cross sectional view of the fourth portion comprised in the valve member according to the inventive swabbable needleless valve.
Figure 8 is a detailed view of the locking member used in the inventive swabbable needleless valve.

The components and portions in the figures are numbered individually, where the numbers refer to the following:
1. Swabbable needleless valve
2. Insert side
3. Exit side
4. Connective region
5. Body
6. Valve member
7. First portion
8. Expansion portion
9. Middle portion
10. Fourth portion
11. Surface
12. Slit
13. Canal
14. Flow cut-off portion
15. Abutment portion
16. Leaning portion
17. Connection portion
18. Locking member

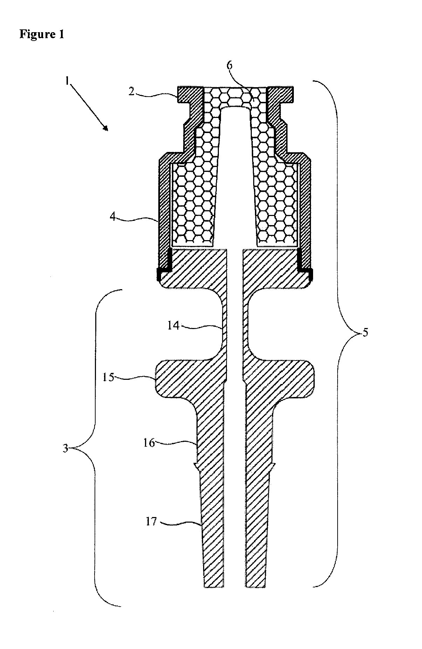

The swabbable needleless valve (1) comprises
a body (5) which has an insert side (2) into which the male luer (M) of the syringe and serum sets used in the treatment are inserted, an exit side (3) which enables to be connected to the catheter and a connective region (4) which connects these two portions (2, 3) to each other, and
a valve member (6) which enables control of the fluid passage. (Figure 1)

The body (5) consists of more than one hollow portions (2, 3, 4) in order to enable flow passage and whose diameters are varied. In a preferred embodiment of the invention, these portions (2, 3, 4) are in the form of cylinder. In the preferred embodiment of the invention, diameter of the connective region (4) is larger than that of the insert side (2) and diameter of the insert side (2) is larger than that of the exit side (3).

The valve member (6) is placed into the cavity provided within the insert side (2) and the connective region (4).

The male luer (M) of the medical device such as sterile syringe, serum set, expansion line which are used to administer medicine and fluid to a patient is inserted into the insert side (2). By inserting said male luer (M), the valve member (6) which is provided in this portion (2) is pushed toward the connective region (4) by being compressed. Depending on the shape of the valve member (6) included, the insert side (2) can consist of more than one portions having the same or different diameters.

The connective region (4) enables connection between the insert side (2) and the exit side (3), and valve member (6) is placed inside the cavity included in the connective region.

The exit side (3) extends towards the catheter from the surface of the connective region (4) opposite to the insert side (2). Said exit side (3) consists of portions which preferably have different geometric features. In the preferred embodiment of the invention, the exit side (3) is composed of four portions; that is, flow cut-off portion (14), abutment portion, (15), leaning portion (16) and connection portion (17). The flow cut-off portion (14) which begins below the connective region (4) has a cross section which firstly gets narrow and then gets wide. Preferably, both ends of the flow cut-off portion (14) have the same cross section and the same geometric shape. The abutment portion (15) which begins from the end of the flow cut-off portion (14) and diameter of which is larger than that of the flow cut-off portion (14) is provided. At the end of the abutment portion (15), the leaning portion (16) and the connection portion (17) begin, respectively. Both of these portions (16, 17) have a constant diameter in the expansion direction and diameter of the leaning portion (16) is larger than that of the connection portion. The leaning portion (16) is leaned to the wall of the catheter to be connected and prevents the catheter from proceeding farther in the exit side. That is, the leaning portion (16) acts as a stopper for the catheter. The connection portion (17) is inserted into the female luer of the catheter, and enables the fluid which passes through the hollow inside of the same to be transmitted to the catheter.

The valve member (6) is placed into the cavity of the insert side (2) and the connective region (4). The valve member (6) which consists of portions having different diameters, has a hole in the middle, one end of which is open and another end of which is close, is in the form of cylinder.

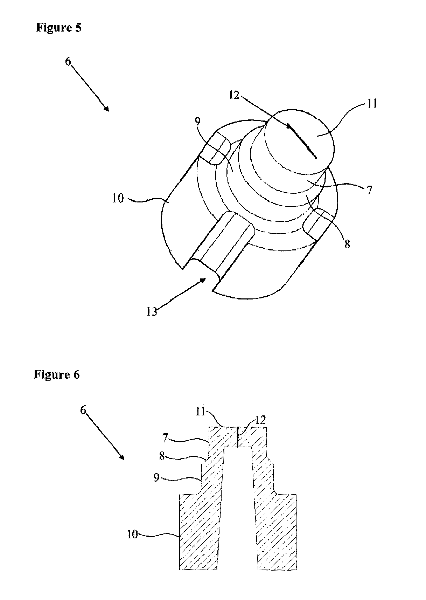

In the preferred embodiment of the invention, the valve member (6) is composed of four sections. In said embodiment, the valve member (6) comprises a first portion (7) which has a surface (11) isolated the valve member (6) from the external environment at the one end and another end of which is hollow, a expansion portion (8) which begins from the open end of the first portion (7) and enables the diameter of the hollow to increase by widening to the outwards, a middle portion (9) which extends below the expansion portion (8) in a constant diameter, and a fourth portion (10) which begins from the end of the middle portion (9), has a larger diameter than that of the middle portion (9) and extends in a constant diameter (Figure 5, Figure 6). Said first portion (7), said expansion portion (8) and said middle portion (9) is placed so as to coincide with the inside of the insert side (2), and the fourth portion (10) is placed so as to coincide with the inside of the connective region (4).

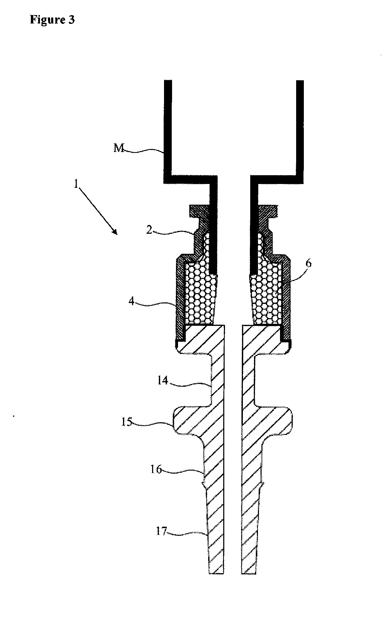

The valve member surface (11) at the end of the first portion (7) facing outside of the swabbable needleless valve (1) comprises at least one slit (12) in order to enable fluid introduction into the body (5). Said slit (12) is in the closed state in a normal position, prevents anything out of the valve (1) from entering into the valve (1). The slit (12) is opened by insertion of the male luer (M) of the medical devices such as sterile syringe, serum set, extension line which are used for administration of medicine and fluid into the insert side (2), and pushing the valve member (6) toward the connective region (4). Thereby, the fluid is enabled to pass to the exit side of the body (5) through the hole in the valve member (6) (Figure 3). When said male luer (M) is removed from the body (5), in the first position, the first portion (7), the expansion portion (8) and the middle portion (9) in the insert side (2) revert to former shape, and the slit (12) on the surface (11) at the end of the first portion (7) is closed.

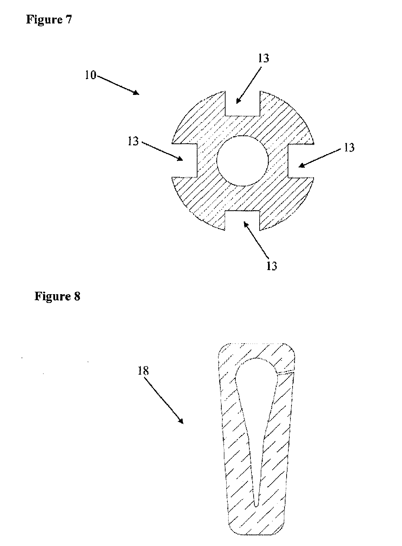

At least one hollow canal (13) is provided on the external surface of the fourth portion (10) which comprises the valve member (6) and preferably has the largest diameter (Figure 7). By means of said canal (13), the first portion (7), expansion portion (8) and middle portion (9) of the valve member (6) are enabled to be displaced to the connective region (4) by the male luer of the medical device inserted into the insert side (2) in order to perform fluid exchange. The displaced valve member portions (7, 8, 9) cause said canal (13) to get narrower by elastic deformation; thereby the luer (M) is seated into the swabbable needleless valve (1) while the required volume is provided.

In the preferred embodiment of the invention, the canal (13) on the external surface of the fourth portion (10) is a vertical canal extending in parallel to the internal hollow of the fourth portion (10).

The surface on the inventive swabbable needleless valve (1) can be cleaned by being wiped. Said surface (11) has a solid flat and smooth structure. Thereby, maximum safety is provided for the patient in the course of the treatment.

The valve member (6) in the inventive swabbable needleless valve (1) is suitable for receiving the male luer (M) of syringes and serum sets used in the treatment. When the luer (M) is removed from the valve member (6), the slit (12) on said valve member (6) is closed, whereby contact with the external environment is cut off.

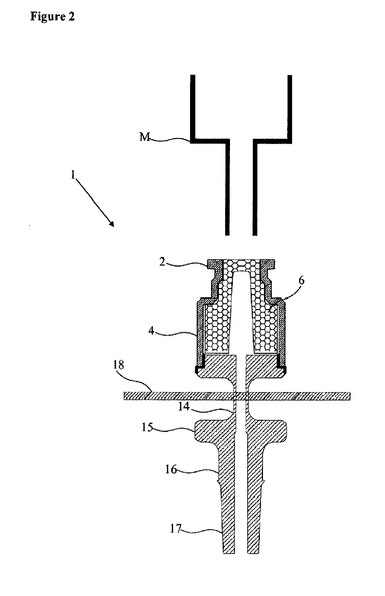

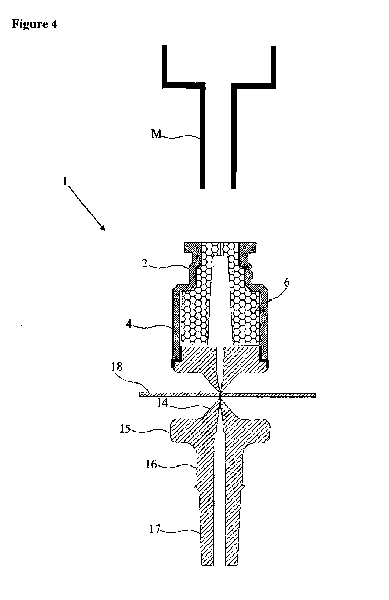

Additionally, in the inventive swabbable needleless valve (1), a locking member (18) is provided between the connective region (4) and the leaning portion (15), on the flow cut-off portion (14). In the preferred embodiment of the invention, the locking member (18) is in the shape of a rectangular plate on which there is a space width of which is getting decreased (Figure 8). In the case wherein the valve member (6) is displaced, the flow cut-off portion (14) stays in the wide part of the space on the locking member (18). Thereby, fluid flow is not occluded (Figure 2). However, when the process of fluid exchange through the catheter is completed, valve member (6) returns back to the extending position while the locking member (18) is slid on the flow cut-off portion (14) and as a result of this sliding process it constrains the walls of the flow cut-off portion (14). As a result, said locking member (18) and side walls of the flow cut-off portion (14) are constrained till the side walls contact with each other and so as not to exist a space therebetween (Figure 4). Thus, the constrained valve member (6) reverts to former shape, that is, due to volume and pressure difference to occur during opening of canals (13) on the fourth portion (10) again, blood flow from the catheter to the valve member (6) is prevented. Thereby, sucking of blood into the fluid passageway and occlusion by later clotting are prevented.

In the preferred embodiment of the invention, the body is in massive form. In this case, the body is preferably made of thermoplastic elastomer or silicon. The body's having massive structure (5) provides molding and assembling simplicity and reduces the cost.

In another embodiment of the invention, the body (5) consists of two different parts. In said embodiment, insert side (2) and the connective portion (4) of the body (5) are manufactured as a single part and the exit side (3) of the same as another part. And then, these parts are connected to each other via the flow cut-off portion (14). In this embodiment, the flow cut-off portion (14) is manufactured from an elastic material such as thermoplastic elastomer, and the members composing the rest of the body is preferably manufactured from hard polycarbonate. Due to elasticity of the flow cut-off portion (14), it can be constrained by means of the locking member (18), whenever required, and thereby fluid flow is occluded.

All of the bodies inspired by the present invention are not disclosed herein. Bodies composing of the invention can show variation in terms of sizes and quantities. For example, the number of the canals (13) included in the valve member (6) can vary from 2 to 10. The geometry of these canals (13) which extend to cavity in the valve member (6) vertically, parallelly or angularly can be in the form of cylinder, semicircular cylinder, elliptical cylinder, square prism, rectangular prism, parallelogram prism, triangular prism, cone, inverted cone.

By means of the inventive swabbable needleless valve (1), the fluid flow pathway is isolated from the external environment, whereby transmission of microorganisms to the vein of a patient is prevented. As the valve (1) is plain and has a limited number of parts, it is manufactured easily, cost effectively and serially. Serum set or syringe tip can be easily inserted into and removed from the valve (1) easily, insert surface (11) of the valve reverts exactly to the original form after every insertion and removal. Due to the fact that the insert surface (11) of the valve (1) which is in contact with the external environment is plain and dead-spaceless, said surface (11) can be disinfected by being wiped easily before the syringe-serum set tip is inserted. As the valve (1) has a fluid flow pathway which is wide, plain-smooth enough and there are not any obstacles such as dead space and joint in the fluid flow pathway, the valve does not restrict the flow by gravity force while the fluid is administered to a patient, and can be connected easily to every type of serum set and syringe tip. The valve (1) reduces variety of devices (cap, needle, latex membrane cap, connector, cock, stopcock) used in the treatment, and enables total cost of the treatment to be reduced.

For better understanding, the inventive "Swabbable Needleless Valve (1)" is described by mean of examples and illustrated by the drawings. However, the scope of the invention cannot be limited to these examples and illustrations. It is possible for a person skilled in the art to realize further embodiments within the scope of the invention. The scope of the invention is essentially according to claims.

## Claims

1. A swabbable needleless valve (1) comprising a body (5) which has an insert side (2) into which the male luer (M) of the syringe and serum sets used in the treatment are inserted, an exit side (3) which enables to be connected to the catheter, and a connective region (4) which connects these two portions (2, 3) to each other, and
**characterized by** a valve member (6) comprising a first portion (7) which realizes fluid flow control and has a surface (11) isolated the valve member (6) from the external environment at the one end and another end of which is hollow, a expansion portion (8) which begins from the open end of the first portion (7) and enables the diameter of the hollow to widen by extending to the outwards, a middle portion (9) which extends below the expansion portion (8) in a constant diameter, and a fourth portion (10) which begins from the end of the middle portion (9), has a larger diameter than the middle portion (9) and extends in a constant diameter.

2. A swabbable needleless valve (1) according to Claim 1, **characterized by** an exit side (3) comprising a flow cut-off portion (14) which begins below the connective region (4) and has a cross section which is firstly getting narrow and then getting wide,
a leaning portion (15) which begins at the end of the flow cut-off portion (14) and diameter of which is larger than that of the flow cut-off portion (14),
an abutment portion (16) which begins at the end of the leaning portion (15), extends in a constant diameter and reclines to the wall of the catheter to be connected and prevents the catheter from proceeding farther in the exit side (3),
a connection portion (17) which begins at the end of the leaning portion (16), extends in a constant diameter and inserts into the female luer of the catheter, and enables the fluid which passes through the hollow inside of the same to be transmitted to the catheter.

3. A swabbable needleless valve (1) according to Claims 1 and 2, **characterized by** a valve member (6) which is placed into the hollow in the insert side (2) and the connective region (4).

4. A swabbable needleless valve (1) according to Claims 1 to 3, **characterized by** a valve member (6) which comprises a first portion (7), an expansion portion (8) and a middle portion (9) placed so as to coincide with the inside of the insert side (2), and the fourth portion (10) placed so as to coincide with the inside of the connective region (4) of the body (5).

5. A valve member (6) according to any of the preceding claims, **characterized by** an insert side (2) having a surface (11) which comprises at least one slit (12) which enables fluid introduction into the body (5).

6. A valve member (6) according to any of the preceding claims, **characterized by** a fourth portion (10) which has at least one hollow canal (13) on its external surface.

7. A fourth portion (10) according to Claim 6, **characterized by** a canal (13) extending in parallel to inner hole of the fourth portion (10) which is in vertical form.

8. A swabbable needleless valve (1) according to any of the preceding claims, **characterized by** at least one locking member (18) which is provided between the connective region (4) and the abutment portion (15), above the flow cut-off portion (14), and is in the shape of a rectangular plate on which there is a space width of which is getting decreased.

9. A swabbable needleless valve (1) according to Claims 1 to 8, **characterized by** a body (5) which has a massive structure.

10. A swabbable needleless valve (1) according to Claim 9, **characterized by** a body (5) which is made of thermoplastic elastomer.

11. A swabbable needleless valve (1) according to Claim 9, **characterized by** a body (5) which is made of silicon.

12. A swabbable needleless valve (1) according to Claims 1 to 8, **characterized by** a body (5) which composed of two different parts one part of which includes the insert side (2) and the connective region (4), and the another part of which includes the exit side (3).

13. A swabbable needleless valve (1) according to Claim 12, **characterized by** a flow cut-off portion (14) which connects two parts to each other.

14. A swabbable needleless valve (1) according to Claims 12 and 13, **characterized by** a flow cut-off portion (14) which is made of thermoplastic elastomer.

15. A swabbable needleless valve (1) according to Claims 12 to 14, **characterized by** a body (5) which has an insert side (2), a connective region (4) and an exit side (3) which are made of solid polycarbonat.
